# EUROPÄISCHE PATENTANMELDUNG

(11) **EP 1 375 601 A1**
(43) Veröffentlichungstag der Anmeldung: **02.01.2004**
(21) Anmeldenummer: 03012193.3
(22) Anmeldetag: 05.06.2003
(51) Int. Cl.: C09C 1/00, C09D 5/36

(54) **Fünfschichtpigmente**

(30) Priorität: 28.06.2002 DE 10229256; 01.11.2002 DE 10251378
(71) Anmelder: MERCK PATENT GmbH, 64293 Darmstadt (DE)
(72) Erfinder: Schmidt, Christoph, 65830 Kriftel (DE); Heyland, Andrea, 64385 Reichelsheim (DE); Schoen, Sabine, 45701 Herten (DE); Hochstein, Veronika, 76646 Bruchsaal (DE); Hensen, Uta, 64283 Darmstadt (DE)

(57) **Zusammenfassung**

Die vorliegende Erfindung betrifft Fünfschichtpigmente auf der Basis von mehrfach beschichteten plättchenförmigen Substraten, die eine Schichtenfolge aus
(A) einer Schicht aus SnO₂ mit einer Schichtdicke von 0,1-50 nm,
(B) einer hochbrechenden Beschichtung bestehend aus TiO₂ in der Rutilmodifikation mit einer Schichtdicke von 10-800 nm,
(C) einer farblosen Beschichtung mit einem Brechungsindex n ≤ 1,8 mit einer Schichtdicke von 20-800 nm,
(D) einer Schicht aus SnO₂ mit einer Schichtdicke von 0,1-50 nm,
(E) einer hochbrechenden Beschichtung bestehend aus TiO₂ in der Rutilmodifikation mit einer Schichtdicke von 10-800 nm,
und optional
(F) einer äußeren Schutzschicht
enthalten,
sowie deren Verwendung in Farben, Lacken, Pulverlacken, Druckfarben, Sicherheitsdruckfarben, Kunststoffen, keramischen Materialien, Gläsern, als Dotierstoff für die Lasermarkierung von Papieren und Kunststoffen, in kosmetischen Formulierungen und zur Herstellung von Pigmentpräparationen und Trockenpräparaten.

## Beschreibung

Die vorliegende Erfindung betrifft Fünfschichtpigmente auf der Basis von mehrfach beschichteten plättchenförmigen Substraten.

Glanz- oder Effektpigmente werden in vielen Bereichen der Technik eingesetzt, insbesondere im Bereich der Autolacke, der dekorativen Beschichtung, im Kunststoff, in Farben, Druckfarben sowie in kosmetischen Formulierungen.

Glanzpigmente, die einen winkelabhängigen Farbwechsel zwischen mehreren Interferenzfarben zeigen, sind aufgrund Ihres Farbenspiels von besonderem Interesse für Autolacke sowie bei fälschungssicheren Wertschriften.

Aus dem Stand der Technik sind Verfahren zur Herstellung von Perlglanzpigmenten bekannt, mit deren Hilfe alternierende Schichten mit hoher und niedriger Brechzahl auf feinteilige Substrate aufgebracht werden können. Derartige Pigmente auf Basis von mehrfach beschichteten plättchenförmigen Substraten sind z.B. aus der U.S. 4,434,010, JP H7-759, U.S. 3,438,796, U.S. 5,135,812, DE 44 05 494, DE 44 37 753, DE 195 16 181 und DE 195 15 988, DE 196 18 565, DE 197 46 067 sowie aus der Literatur z.B. aus EURO COSMETICS, 1999, Nr. 8, S. 284 bekannt.

Besondere Bedeutung besitzen dabei Perlglanzpigmente auf mineralischer Basis. Perlglanzpigmente werden durch Beschichtung eines anorganischen, plättchenförmigen Trägers mit einer hochbrechenden, meist oxidischen Schicht hergestellt. Die Farbe dieser Pigmente wird durch wellenlängenselektive Teilreflektion und Interferenz des reflektierten bzw. transmittierten Lichts an den Medium/Oxid- bzw. Oxid/Substrat-Grenzflächen hervorgerufen.

Die Interferenzfarbe dieser Pigmente wird von der Dicke der Oxidschicht bestimmt. Der Farbton eines Interferenzsilberpigments wird durch eine im optischen Sinn einzelne hochbrechende Schicht erzeugt, deren optische Dicke ein Reflexionsmaximum (1. Ordnung) im sichtbaren Wellenlängenbereich bei ca. 500 nm bedingt. Diese Wellenlänge nimmt das menschliche Auge als die Farbe Grün wahr. Der Intensitätsverlauf dieses Maximums auf deren Wellenlängenachse ist jedoch so breit, dass im ganzen Bereich des sichtbaren Lichts so viel Licht reflektiert wird, dass das menschliche Auge einen sehr hellen, aber farblosen Eindruck wahrnimmt.

Gemäß den - insbesondere aus der Vergütung optischer Bauteile - bekannten Regeln der Optik dünner Schichten steigt im Vergleich zum Einschichtsystem die Intensität am Interferenzmaximum um mehr als 60 % an. Demzufolge wird das Profil des durch Interferenz reflektierten Lichts wesentlich ausgeprägter, so dass für ein solches mehrschichtiges System eine grüne Reflexionsfarbe zu erwarten ist.

Überraschenderweise wurden nun Fünfschichtpigmente gefunden, die bezüglich ihrer koloristischen aber auch ihrer anwendungstechnischen Eigenschaften deutlich verbesserte Eigenschaften zeigen als die Mehrschichtpigmente aus dem Stand der Technik. Dies konnte durch eine innere Strukturierung der hochbrechenden Schichten realisiert werden.

Gegenstand der Erfindung sind daher Fünfschichtpigmente auf der Basis von mehrfach beschichteten plättchenförmigen Substraten, die mindestens eine Schichtenfolge aus
(A) einer Schicht aus SnO₂ mit einer Schichtdicke von 0,1-50 nm,
(B) einer Schicht aus TiO₂ in der Rutilmodifikation mit einer Schichtdicke von 10-800 nm,
(C) einer farblosen Beschichtung mit einem Brechungsindex n≤ 1,8 mit einer Schichtdicke von 20-800 nm,
(D) einer Schicht aus SnO₂ mit einer Schichtdicke von 0,1-50 nm
(E) einer Schicht aus TiO₂ in der Rutilmodifikation mit einer Schichtdicke von 10-800 nm,
   und optional
(F) einer äußeren Schutzschicht
enthalten.

Die erfindungsgemäßen Mehrschichtpigmente sind Interferenzpigmente, die gegenüber den bekannten Mehrschichtpigmenten mit drei Schichten
- eine deutlich erhöhte Helligkeit
- einen stärkeren Glanz und
- einen stärker ausgeprägten Farbflop
aufweisen.

Gegenstand der Erfindung ist weiterhin die Verwendung der erfindungsgemäßen Mehrschichtpigmente in Farben, Lacken, Pulverlacken, Druckfarben, Kunststoffen, keramischen Materialien, Gläsern und kosmetischen Formulierungen, insbesondere in der dekorativen Kosmetik. Weiterhin sind die erfindungsgemäßen Pigmente auch zur Herstellung von Pigmentpräparationen sowie zur Herstellung von Trockenpräparaten, wie z.B. Granulaten, Chips, Pellets, Briketts, etc. geeignet. Die Trockenpräparate sind insbesondere für Druckfarben geeignet.

Geeignete Basissubstrate für die erfindungsgemäßen Mehrschichtpigmente sind farblose oder selektiv oder nicht selektiv absorbierende plättchenförmige Substrate. Bevorzugte Substrate sind Schichtsilikate. Insbesondere geeignet sind natürlicher und/oder synthetischer Glimmer, Talkum, Kaolin, plättchenförmige Eisen- oder Aluminiumoxide, Glas-, SiO₂-, TiO₂-, Graphitplättchen, synthetische trägerfreie Plättchen, Titannitrid, Titansilicid, Liquid crystal polymers (LCPs), holographische Pigmente, BiOCI, plättchenförmige Mischoxide, wie z.B. FeTiO₃, Fe₂TiO₅ oder andere vergleichbare Materialien.

Die Größe der Basissubstrate ist an sich nicht kritisch und kann auf den jeweiligen Anwendungszweck abgestimmt werden. In der Regel haben die plättchenförmigen Substrate eine Dicke zwischen 0,005 und 10 µm, insbesondere zwischen 0,05 und 5 µm. Die Ausdehnung in den beiden anderen Bereichen beträgt üblicherweise zwischen 1 und 500 µm, vorzugsweise zwischen 2 und 200 µm und insbesondere zwischen 5 und 60 µm.

Die Dicke der Schicht (A) bzw. (D) beträgt 0,1-50 nm, vorzugsweise 0,3-30 nm, insbesondere 0,5-20 nm. Die SnO₂-Schichten (A) und (D) können gleiche oder unterschiedliche Schichtdicken aufweisen.

Die Dicke der einzelnen Schichten (B) und (C) und (E) mit hohem bzw. niedrigem Brechungsindex auf dem Basissubstrat ist wesentlich für die optischen Eigenschaften des Pigments. Für das erfindungsgemäße Mehrschichtpigment müssen die Dicken der einzelnen Schichten genau aufeinander eingestellt werden.

Die Dicke der Schicht (B) bzw. (E) beträgt 10-800 nm, vorzugsweise 20-500 nm, insbesondere 30-400 nm. Die TiO₂-Schichten (B) und (E) können gleiche oder unterschiedliche Schichtdicken aufweisen. Die Dicke der Schicht (C) beträgt 20-800 nm, vorzugsweise 30-600 nm, insbesondere 40-500 nm.

Als farblose niedrigbrechende für die Beschichtung (C) geeignete Materialien sind vorzugsweise Metalloxide bzw. die entsprechenden Oxidhydrate, wie z.B. SiO₂, Al₂O₃, AIO(OH), B₂O₃, MgF₂, MgSiO₃ oder ein Gemisch der genannten Metalloxide, geeignet. Bei der Schicht (C) handelt es sich insbesondere um eine SiO₂-Schicht.

Die erfindungsgemäßen Fünfschichtpigmente lassen sich herstellen durch die Erzeugung mehrerer hoch- und niedrigbrechender Interferenzschichten mit genau definierter Dicke und glatter Oberfläche auf den feinteiligen, plättchenförmigen Substraten.

Die Metalloxidschichten werden vorzugsweise nasschemisch aufgebracht, wobei die zur Herstellung von Perlglanzpigmenten entwickelten nasschemischen Beschichtungsverfahren angewendet werden können. Derartige Verfahren sind z.B. beschrieben in DE 14 67 468, DE 19 59 988, DE 20 09 566, DE 22 14 545, DE 22 15 191, DE 22 44, 298, DE 23 13 331, DE 15 22 572, DE 31 37 808, DE 31 37 809, DE 31 51 343, DE 31 51 354, DE 31 51 355, DE 32 11 602, DE 32 35 017 oder auch in weiteren dem Fachmann bekannten Patentdokumenten und sonstigen Publikationen.

Bei der Nassbeschichtung werden die Substratpartikel in Wasser suspendiert und mit ein oder mehreren hydrolysierbaren Metallsalzen oder einer Wasserglaslösung bei einem für die Hydrolyse geeigneten pH-Wert versetzt, der so gewählt wird, dass die Metalloxide bzw. Metalloxidhydrate direkt auf den Plättchen ausgefällt werden, ohne dass es zu Nebenfällungen kommt. Der pH-Wert wird üblicherweise durch gleichzeitiges Zudosieren einer Base und/oder Säure konstant gehalten. Anschließend werden die Pigmente abgetrennt, gewaschen und bei 50-150 °C für 6-18 h getrocknet und gegebenenfalls 0,5-3 h geglüht, wobei die Glühtemperatur im Hinblick auf die jeweils vorliegende Beschichtung optimiert werden kann. In der Regel liegen die Glühtemperaturen zwischen 250 und 1000 °C, vorzugsweise zwischen 350 und 900 °C. Falls gewünscht, können die Pigmente nach Aufbringen einzelner Beschichtungen abgetrennt, getrocknet und ggf. geglüht werden, um dann zur Auffüllung der weiteren Schichten wieder resuspendiert zu werden.

Weiterhin kann die Beschichtung auch in einem Wirbelbettreaktor durch Gasphasenbeschichtung erfolgen, wobei z.B. die in EP 0 045 851 und EP 0 106 235 zur Herstellung von Perlglanzpigmenten vorgeschlagenen Verfahren entsprechend angewendet werden können.

Der Farbton der Interferenzpigmente kann in sehr weiten Grenzen durch unterschiedliche Wahl der Belegungsmengen bzw. der daraus resultierenden Schichtdicken variiert werden. Die Feinabstimmung für einen bestimmten Farbton kann über die reine Mengenwahl hinaus durch visuell oder messtechnisch kontrolliertes Anfahren der gewünschten Farbe erreicht werden.

Zur Erhöhung der Licht-, Wasser- und Wetterstabilität empfiehlt es sich häufig, in Abhängigkeit vom Einsatzgebiet, das fertige Pigment einer Nachbeschichtung oder Nachbehandlung zu unterziehen. Als Nachbeschichtungen bzw. Nachbehandlungen kommen beispielsweise die in den DE-PS 22 15 191, DE-OS 31 51 354, DE-OS 32 35 017 oder DE-OS 33 34 598 beschriebenen Verfahren in Frage. Durch diese Nachbeschichtung (Schicht F) wird die chemische Stabilität weiter erhöht oder die Handhabung des Pigments, insbesondere die Einarbeitung in unterschiedliche Medien, erleichtert.

Die erfindungsgemäßen Pigmente sind mit einer Vielzahl von Farbsystemen kompatibel, vorzugsweise aus dem Bereich der Lacke, Farben und Druckfarben. Für die Herstellung der Druckfarben für, z.B. den Tiefdruck, Flexodruck, Offsetdruck, Offsetüberdrucklackierung, ist eine Vielzahl von Bindern, insbesondere wasserlösliche Typen, geeignet, wie sie z.B. von den Firmen BASF, Marabu, Pröll, Sericol, Hartmann, Gebr. Schmidt, Sicpa, Aarberg, Siegberg, GSB-Wahl, Follmann, Ruco oder Coates Screen INKS GmbH vertrieben werden. Die Druckfarben können auf Wasserbasis oder Lösemittelbasis aufgebaut sein. Weiterhin sind die Pigmente auch für die Lasermarkierung von Papier und Kunststoffen sowie für die Anwendungen im Agrarbereich, z.B. für Gewächshausfolien sowie z.B. für die Farbgebung von Zeltplanen, geeignet.

Da die erfindungsgemäßen Mehrschichtpigmente starken Glanz mit hoher Helligkeit, Transparenz und stark ausgeprägtem Farbflop verbinden, lassen sich mit ihnen besonders wirksame Effekte in den verschiedenen Anwendungsmedien erzielen, z.B. in kosmetischen Formulierungen wie z.B. Nagellacken, Lippenstiften, Presspudern, Gelen, Lotionen, Seifen, Zahnpasten, in Lacken wie z.B. Autolacken, Industrielacken und Pulverlacken sowie in Kunststoffen und in der Keramik.

Aufgrund des guten Skin Feelings und der sehr guten Hautadhäsion sind die erfindungsgemäßen Pigmente insbesondere für die dekorative Kosmetik geeignet.

Es versteht sich von selbst, dass für die verschiedenen Anwendungszwecke die erfindungsgemäßen Mehrschichtpigmente auch vorteilhaft in Abmischung mit organischen Farbstoffen, organischen Pigmenten oder anderen Pigmenten, wie z.B. transparenten und deckenden Weiß-, Buntund Schwarzpigmenten sowie mit plättchenförmigen Eisenoxiden, organischen Pigmenten, holographischen Pigmenten, LCPs (Liquid Crystal Polymers) und herkömmlichen transparenten, bunten und schwarzen Glanzpigmenten auf der Basis von metalloxidbeschichteten Glimmer- und SiO₂-Plättchen etc. verwendet werden können. Die erfindungsgemäßen Fünfschichtpigmente können in jedem Verhältnis mit handelsüblichen Pigmenten und Füllern gemischt werden.

Als Füllstoffe sind z.B. zu nennen natürlicher und synthetischer Glimmer, Nylon Powder, reine oder gefüllte Melaninharze, Talcum, Gläser, Kaolin, Oxide oder Hydroxide von Aluminium, Magnesium, Calcium, Zink, BiOCI, Bariumsulfat, Calciumsulfat, Calciumcarbonat, Magnesiumcarbonat, Kohlenstoff, sowie physikalische oder chemische Kombinationen dieser Stoffe.

Bezüglich der Partikelform des Füllstoffes gibt es keine Einschränkungen. Sie kann den Anforderungen gemäß z.B. plättchenförmig, sphärisch oder nadelförmig sein.

Selbstverständlich können die erfindungsgemäßen Pigmente in den Formulierungen auch mit jeder Art von kosmetischen Roh- und Hilfsstoffen kombiniert werden. Dazu gehören u.a. Öle, Fette, Wachse, Filmbildner, Konservierungsmittel und allgemein anwendungstechnische Eigenschaften bestimmende Hilfsstoffe, wie z.B. Verdicker und rheologische Zusatzstoffe wie etwa Bentonite, Hektorite, Siliciumdioxide, Ca-Silicate, Gelatine, hochmolekulare Kohlenhydrate und/oder oberflächenaktive Hilfsmittel etc.

Die die erfindungsgemäßen Pigmente enthaltenden Formulierungen können dem lipophilen, hydrophilen oder hydrophoben Typ angehören. Bei heterogenen Formulierungen mit diskreten wäßrigen und nichtwässrigen Phasen können die erfindungsgemäßen Pigmente in jeweils nur einer der beiden Phasen enthalten oder auch über beide Phasen verteilt sein.

Die pH-Werte der Formulierungen können zwischen 1 und 14, bevorzugt zwischen 2 und 11 und besonders bevorzugt zwischen 5 und 8 liegen.

Den Konzentrationen der erfindungsgemäßen Pigmente in der Formulierung sind keine Grenzen gesetzt. Sie können - je nach Anwendungsfall - zwischen 0,001 (rinse-off-Produkte, z.B. Duschgele) - 100 % (z.B. Glanzeffekt-Artikel für besondere Anwendungen) liegen.

Die erfindungsgemäßen Pigmente können weiterhin auch mit kosmetischen Wirkstoffen kombiniert werden. Geeignete Wirkstoffe sind z.B. Insect Repellents, UV A/BC-Schutzfilter (z.B. OMC, B3, MBC), Anti-Ageing-Wirkstoffe, Vitamine und deren Derivate (z.B. Vitamin A, C, E etc.), Selbstbräuner (z.B. DHA, Erytrolose u.a.) sowie weitere kosmetische Wirkstoffe wie z.B. Bisabolol, LPO, Ectoin, Emblica, Allantoin, Bioflavanoide und deren Derivate.

Die erfindungsgemäßen Pigmente sind weiterhin geeignet zur Herstellung von fließfähigen Pigmentpräparationen und Trockenpräparaten enthaltend ein oder mehrere erfindungsgemäße Pigmente, Bindemittel und optional ein oder mehrere Additive. Unter Trockenpräparate sind auch Präparate zu verstehen, die 0 bis 8 Gew.% an Wasser und/oder eines Lösemittels oder Lösemittelgemisches enthalten. Die Trockenpräparate liegen vorzugsweise als Pellets, Granulate, Chips und Briketts vor. Vorzugsweise werden die Trockenpräparate bei der Herstellung von Druckfarben und in kosmetischen Formulierungen eingesetzt.

Gegenstand der Erfindung ist weiterhin auch die Verwendung der Pigmente in Formulierungen wie Farben, Druckfarben, Sicherheitsdruckfarben, Lacken, Kunststoffen, keramischen Materialien, Gläsern, in kosmetischen Formulierungen, als Dotierstoff für die Lasermarkierung von Papieren und Kunststoffen sowie zur Herstellung von Pigmentpräparationen und Trockenpräparaten.

Die nachfolgenden Beispiele sollen die Erfindung näher erläutern ohne sie jedoch zu beschränken.

### Beispiele

### Beispiel 1: 5-Schichtpigment mit ausgeprägtem Farbflop

100 g Glimmer der Teilchengröße 10 - 60 µm werden in 2 l entmineralisiertem Wasser unter Rühren auf 80 °C erhitzt. Nach Erreichen dieser Temperatur wird bei pH 1,8 unter Rühren eine Lösung bestehend aus 2,3 g SnCl₄ x 5 H₂O und 10 ml Salzsäure (37 %) in 70 ml VE-Wasser langsam zu der Glimmersuspension zudosiert. Danach wird bei einem pH-Wert von 1,8 220 g einer 32%igen TiCl₄-Lösung (400 g TiCl₄/l) zudosiert, wobei der pH-Wert mit 32%iger Natronlauge konstant gehalten wird. Anschließend wird der pH-Wert auf 7,5 angehoben und bei diesem pH-Wert werden 800 ml Natronwasserglaslösung (13,0 Gew. % SiO₂) langsam zudosiert, wobei der pH-Wert mit 10%iger HCI auf 7,5 konstant gehalten wird. Nach einer Nachrührzeit von ca. 0,5 h wird der pH-Wert mit Salzsäure (10 %) auf pH 1,8 abgesenkt und eine Lösung von 2,3 g SnCl₄ x 5 H₂O und 10 ml Salzsäure (32 %) in 70 ml VE-Wasser zudosiert. Anschließend werden bei einem pH-Wert von 1,8 ca. 200 ml TiCl₄-Lösung (400 g/l TiCl₄) langsam zudosiert. Der pH-Wert wird jeweils mit Natronlauge (32 Gew. %) konstant gehalten. Nach einer weiteren Nachrührzeit von 0,5 h bei pH=1,8 wird das beschichtete Glimmerpigment abfiltriert, gewaschen und bei 110 °C 16 h getrocknet. Zuletzt wird das Pigment 0,5 h bei 850 °C geglüht.

Man erhält ein blaues Interferenzpigment mit scharfem Farbwechsel von Blau nach Rot beim Übergang von steilem zu flachem Beobachtungswinkel.

### Beispiel 2

100 g Glimmer der Teilchengröße 10 - 60 µm werden in 2 l entmineralisiertem Wasser unter Rühren auf 80 °C erhitzt. Nach Erreichen dieser Temperatur wird unter starkem Rühren eine Lösung bestehend aus 3 g SnCl₄ x 5 H₂O und 10 ml Salzsäure (37 %) in 90 g Wasser langsam zu der Glimmersuspension zudosiert. Bei einem pH-Wert von 1,8 werden 220 ml TiCl₄-Lösung (400 g TiCl₄/l) zudosiert. Danach wird der pH-Wert konstant auf 7,5 gehalten. Anschließend wird der pH-Wert auf 7,5 angehoben und bei diesem pH-Wert werden 830 ml Natronwasserglaslösung (13 Gew. % SiO₂) langsam zudosiert, wobei der pH-Wert auf 10%iger HCI auf 7,5 konstant gehalten wird. Anschließend werden bei pH=1,8 eine Lösung von 11,5 g SnCl₄ x 5 H₂O und 10 ml Salzsäure (32 %) in 350 ml VE-Wasser zudosiert. Zuletzt werden bei pH=1,8 220 ml TiCl₄-Lösung (400 g TiCl₄/l) zudosiert. Der pH-Wert wird bei der Zugabe der SnCl₄ x 5 H₂O-Lösungen und TiCl₄-Lösungen jeweils mit NaOH-Lösung (32 %) konstant gehalten.

Zur Aufarbeitung wird das beschichtete Glimmerpigment abfiltriert, gewaschen und bei 110 °C 16 h getrocknet. Zuletzt wird das Pigment 0,5 h bei 850 °C geglüht.

Man erhält ein hellglänzendes grünblaues Interferenzpigment, dessen Farbe bei Beobachtung im flachen Winkel rotviolett ist.

### Anwendunqsbeispiele

### Beispiel A1: Wet Lip Balm mit 10 % Perlglanzpigment

| Phase A | | |
|---|---|---|
| Fünfschichtpigment | Silica, Cl77891 (Titanium dioxide), | |
| gemäß Beispiel 1 | Mica, Tin oxide | 10,00% (1) |

| Phase B | | |
|---|---|---|
| Vegelatum Equiline EU103 | | 11,00 % (2) |
| Candelilla Wax | Candelilla Cera(Candelilla Wax) | 7,00 % (3) |
| Bienenwachs gebleicht | Cera Alba(Beeswax) | 5,00 % (1) |
| Myritol 331 | Cocoglycerides | 8,00 % (4) |
| Ozokerite Wax White # 77W | Ozokerite | 3,00 % (3) |
| Isopropylmyristat | Isopropyl Myristate | 5,00 % (4) |
| Eusolex® 2292 | Ethylhexyl Methoxycinnamate, BHT | 7,00 % (1) |
| Eusolex® OCR | Octocrylene | 4,00 % (1) |
| Oxynex® K flüssig | PEG-8, Tocopherol, Ascorbyl Palmitate, | |
| | Ascorbic Acid, Citric Acid | 0,10 % (1) |
| Propyl-4-hydroxybenzoat | Propylparaben | 0,10 % (1) |
| Farbdispersion in Rizinusöl | | 0,50 % |
| Rizinusöl | Ricinus Communis (Castor Oil) | 38,80 % (5) |

| Phase C | | |
|---|---|---|
| Parfümöl Tendresse # 75418C | Parfum | 0,50 % (6) |

### Herstellung:

Die Bestandteile der Phase B werden unter Rühren auf 75 °C erhitzt bis alles geschmolzen ist. Phase A zugeben und gut durchrühren. Die Lippenstiftmasse dann in der auf 65 °C temperierten Gießapparatur füllen, Phase C zugeben und 15 Minuten rühren. Die homogene Schmelze wird in die auf 55 °C vorgewärmten Gießform gegossen.

Anschließend kühlt man die Formen ab und entfernt die Gießlinge kalt. Nach Erwärmen der Lippenstifte auf Raumtemperatur werden die Lippenstifte kurz abgeflammt.

### Bezugsauellen:

(1) Merck KGaA/Rona®
(2) Natunola Health Inc.
(3) Ross Waxes
(4) Cognis GmbH
(5) Henry Lamotte GmbH
(6) Haarmann & Reimer GmbH

### Beispiel A2: Duschgel

| Phase A | | |
|---|---|---|
| Fünfschichtpigment | Silica, Cl77891 (Titanium dioxide), | |
| gemäß Beispiel 1 | Mica,Tin oxide | 0,10 % (1) |
| Keltrol T | Xanthan Gum | 0,75 % (2) |
| Wasser, demineralisiert | Aqua (Water) | 64,95 % |

| Phase B | | |
|---|---|---|
| Plantacare 2000 UP | Decyl Glucoside | 20,00 % (3) |
| Texapon ASV 50 | Sodium Laureth Sulfate, Sodium Laureth-8 Sulfate, Magnesium Laureth Sulfate, Magnesium Laureth-8 Sulfate, Sodium Oleth Sulfate, Magnesium Oleth Sulfate | 3,60 % (3) |
| Bronidox L | Propylene Glycol, 5-Bromo-5-Nitro-1,3-Dioxane | 0,20 % (3) |
| Parfümöl Everest 79658 SB (gestrichen) | parfum | 0,05 % (4) |
| 1 % FD&C Blue No.1 in Wasser | Aqua(Water), Cl 42090 (FD&C Blue No. 1) | 0,20 % (5) |

| Phase C | | |
|---|---|---|
| Citronensäure Monohydrat | Citric Acid | 0,15 % (1) |
| Wasser, demineralisiert | Aqua(Water) | 10,00 % |

### Herstellung:

Für Phase A wird das Fünfschichtpigment in Wasser einrührt. Keltrol T unter Rühren langsam einstreuen und rühren bis es gelöst ist. Die Phasen B und C nacheinander hinzufügen und dabei langsam rühren bis alles homogen verteilt ist. Den pH-Wert auf 6,0 bis 6,5 einstellen.

### Bezugsquellen:

(1) Merck KGaA/Rona®
(2) Kelco
(3) Cognis GmbH
(4) Haarmann & Reimer GmbH
(5) BASF AG

### Beispiel A3: Eyeliner Gel

| Phase A | | |
|---|---|---|
| Fünfschichtpigment | Silica, Cl77891 (Titanium dioxide), | |
| gemäß Beispiel 1 | Mica,Tin oxide | 15,00 % (1) |
| Mica Black | Cl 77499 (Iron Oxides), Mica, Cl 77891 (Titanium Dioxide) | 5,00 % (1) |
| Ronasphere® | Silica | 2,00 % (1) |
| Carbopol ETD 2001 | Carbomer | 0,40 % (2) |
| Citronensäure Monohydrat | Citric Acid | 0,00 % (1) |
| Wasser, demineralisiert | | 60,00 % |

| Phase B | | |
|---|---|---|
| Glycerin, wasserfrei | Glycerin | 4,00 % (1) |
| Triethanolamin reinst | Triethanolamine | 0,90 % (1) |
| Luviskol VA 64 Powder | PVP/VA copolymer | 2,00 % (3) |
| Germaben II | Propylene Glycol, Diazolidinyl Urea, Methylparaben, Propylparaben | 1,00 % (4) |
| Wasser, demineralisiert | Aqua(Water) | 9,70 % |

### Herstellung:

Fünfschichtpigment und Ronasphere® werden im Wasser der Phase A dispergiert. Mit einigen Tropfen Citronensäure ansäuern um die Viskosität zu vermindern, Carbopol unter Rühren einstreuen. Nach vollständiger Lösung die vorgelöste Phase B langsam einrühren und pH-Wert auf 7,0 - 7,5 einstellen.

### Bezugsguellen:

(1) Merck KGaA/Rona®
(2) BF Goodrich
(3) BASF AG
(4) ISP Global Technologies

### Beispiel A4: Lidschatten

| Phase A | | |
|---|---|---|
| Fünfschichtpigment | Silica, Cl77891 (Titanium dioxide), | |
| gemäß Beispiel 2 | Mica,Tin oxide | 55,0 % (1) |
| Biron® B 50 | Cl 77163 (Bismuth Oxychloride) | 3,00 **%** (1) |
| Colorona® Dark Blue | Perlglanzpigment: Mica, Cl 77891 (Titanium Dioxide). Cl 77510 (Ferric Ferrocyanide) | 10,00% (1) |
| Magnesiumstearat | Magnesium Stearate | 2,50 % (1) |
| Weißer Ton (gestrichen) | Kaolin | 5,00 % (1) |
| Hubersorb 600 | Calcium Silicate | 0,50 % (2) |
| Talkum | Talc | 11,00% (1) |

| Phase B | | |
|---|---|---|
| Amerchol L 101 | Lanolin Alcohol, Paraffinum Liquidum | |
| | (Mineral Oil) | 10,70 % (3) |
| Super Hartolan | Lanolin Alcohol | 1,00 % (4) |
| Ewalin 1751 | Petrolatum | 1,00 % (5) |
| Propyl-4-hydroxybenzoat | Propylparaben | 0,10 % (1) |
| Parfümöl Elegance # 79228 D MD | Parfum | 0,20 % (6) |

### Herstellung:

Bestandteile der Phase A zusammengeben und vormischen. Anschließend die Pudermischung unter Rühren tropfenweise mit der geschmolzenen Phase B versetzen. Die Puder werden bei 40 - 50 bar gepresst.

### Bezugsquellen:

(1) Merck KGaA/Rona®
(2) J. M. Huber Corp.
(3) Amerchol
(4) Croda GmbH
(5) H. Erhard Wagner GmbH
(6) Haarmann & Reimer GmbH

### Beispiel A5: Eye Shadow Gel

| Phase A | | |
|---|---|---|
| Fünfschichtpigment | Silica, Cl77891 (Titanium dioxide), | |
| gemäß Beispiel 1 | Mica,Tin oxide | 15,00 % (1) |
| Mica Black | Cl 77499 (Iron oxides), Mica, Cl 77891 (Titanium Dioxide) | 5,00 % (1) |
| Ronasphere® | Silica | 3,00 % (1) |
| Carbopol ETD 2001 | Carbomer | 3,00 % (2) |
| Citronensäure Monohydrat | Citric Acid | 0,00 % (1) |
| Wasser, demineralisiert | Aqua(Water) | 60,00 % |

| Phase B | | |
|---|---|---|
| Glycerin, wasserfrei | Glycerin | 2,00 % (1) |
| Germaben II | Propylene Glycol, Diazolidinyl Urea, Methylparaben, Propylparaben | 0,20 % (3) |
| Triethanolamin reinst | Triethanolamine | 0,70 % (1) |
| Wasser, demineralisiert | Aqua (Water) | 13,80 % |

### Herstellung:

Fünfschichtpigment und Ronasphere® werden im Wasser der Phase A dispergiert. Mit einigen Tropfen Citronensäure ansäuern um die Viskosität zu vermindern, Carbopol unter Rühren einstreuen. Nach vollständiger Lösung die vorgelöste Phase B langsam einrühen.

### Bezugsauellen:

(1) Merck KGaA
(2) BF Goodrich GmbH
(3) ISP Global Technologies

### Beispiel A6: Shimmering Foundation

| Phase A | | |
|---|---|---|
| Extender W | Mica, Cl 77891 (Titanium Dioxide) | 9,00 % (1) |
| Microna® Matte Yellow | Mica, Cl 77492 (Iron Oxides) | 4,00 % (1) |
| Microna® Matte Red | Cl 77491 (Iron Oxides), Mica | 0,40 % (1) |
| Microna® Matte Black | Cl 77499 (Iron Oxides), Mica | 0,30 % (1) |
| Fünfschichtpigment | Silica, Cl77891 (Titanium dioxide), | |
| gemäß Beispiel 1 | Mica, Tin oxide | 4,50 % (1) |
| Ronasphere® | Silica | 5,00 % (1) |

| Phase B | | |
|---|---|---|
| Blanose 7 HF | Cellulose Gum | 0,20 % (2) |
| Veegum | Magnesium Aluminium Silicate | 1 ,00 % (3) |
| Texapon K 1296 | Sodium Lauryl Sulfate | 0,60 % (4) |
| Triethanolamin reinst | Triethanolamine | 0,50 % (1) |
| Titriplex III | Disodium EDTA | 0,25 % (1) |
| Methyl -4-hydroxybenzoat | Methylparaben | 0,15 % (1) |
| 1,2-Propandiol | Propylene Glycol | 10,90% (1) |
| Wasser, demineralisiert | Aqua (Water) | 42,95 % |

| Phase C | | |
|---|---|---|
| Isopropylmyristat | Isopropyl Myristate | 8,00 % (4) |
| Paraffin dünnflüssig | Paraffinum Liquidum (Mineral Oil) | 3,60 % (1) |
| Crodamol SS | Cetyl Esters | 2,60 % (5) |
| Monomuls 60-35 C | Hydrogenated Palm Glycerides | 1,70 % (4) |
| Stearinsäure | Stearic Acid | 1,50 % (1) |
| Eusolex® 6300 | 4-Methylbenzylidene Camphor | 1,30 % (1) |
| Eusolex® 4360 | Benzophenone-3 | 0,50 % (1) |
| RonaCare™ Tocopherolacetat | Tocopheryl Acetate | 0,10 % (1) |
| Magnesiumstearat | Magnesium Stearate | 0,10 % (1) |
| Propyl-4-hydroxybenzoat | Propylparaben | 0,05 % (1) |

| Phase D | | |
|---|---|---|
| Parfümöl 200 529 | Parfum | 0,20 % (6) |
| Euxyl K 400 | Phenoxyethanol, Methyldibromo-Glutaronitrile | 0,20 % (7) |

### Herstellung:

Alle Bestandteile der Phase C bei ca. 75 °C aufschmelzen und rühren, bis alles geschmolzen ist. Das Wasser der Phase B kalt vorlegen, Blanose mit dem Turrax einhomogenisieren, Veegum einstreuen und erneut homogenisieren. Auf 75 °C erwärmen und unter Rühren die übrigen Bestandteile darin lösen. Inhaltsstoffe der Phase A einrühren. Bei 75 °C unter Rühren die Phase C zugeben und 2 min homogenisieren. Die Masse unter Rühren auf 40 °C abkühlen, Phase D zugeben. Unter Rühren auf Raumtemperatur weiter abkühlen und auf pH 6,0 - 6,5 einstellen (z B. mit Citronensäurelösung).

### Bezugsguellen:

(1) Merck KGaA/Rona®
(2) Aqualon GmbH
(3) Vanderbilt
(4) Cognis GmbH
(5) Croda GmbH
(6) Fragrance Resources
(7) Schülke & Mayr GmbH

### Beispiel A7: Lip Gloss

| Phase A | | |
|---|---|---|
| Fünfschichtpigment | Silica, Cl77891 (Titanium dioxide), | |
| gemäß Beispiel 2 | Mica,Tin oxide | 10,00 % (1) |

| Phase B | | |
|---|---|---|
| Indopol H 100 | Polybutene | 59,90 % (2) |
| Bentone Gel MIO V | Quaternium-18 Hectorite, Propylene Carbonate, Paraffinum Liquidum (Mineral Oil) | 20,00 % (3) |
| Eutanol G | Octyldodecanol | 6,00 % (4) |
| RonaCare™ Tocopherolacetat | Tocopheryl Acetate | 1,00% (1) |
| Dow Coming 1403 Fluid | Dimethiconol, Dimethicone | 3,00 % (5) |
| Propyl-4-hydroxybenzoat | Propylparaben | 0,05 % (1) |
| Rouge Covapate W 3773 | Ricinus Communis (Castor Oil), Cl 15850 (D&C) Red No. 6) 0,05 % (6) | |

### Herstellung:

Alle Bestandteile der Phase B werden zusammen eingewogen, auf 70 °C erhitzt und gut durchgerührt bis eine homogene Masse entstanden ist. Dann werden die Pigmente zugegeben und nochmals durchgerührt. Die homogene Mischung füllt man bei 50 - 60 °C ab.

### Bezugsguellen:

(1) Merck KGaA/Rona®
(2) BP Amoco
(3) Elementis Specialites
(4) Cognis GmbH
(5) Dow Corning
(6) Les Colorants Wackherr

### Beispiel A8: Lidschatten - Kompaktpuder

| Phase A | | |
|---|---|---|
| Fünfschichtpigment | Silica, Cl77891 (Titanium dioxide), | |
| gemäß Beispiel 1 | Mica,Tin oxide | 25,00 % (1) |
| Colorona® Dark Blue | Mica, Cl 77891 (Titanium Dioxide), Cl 77510 (Ferric Ferrocyanide) | 5,00 % (1) |
| Talkum | Talc | 49,50 % (1) |
| Kartoffelstärke | Solanium Tuberosum (Potato Starch) | 7,50 % (2) |
| Magnesiumstearat | Magnesium Sterate | 2,50 % (1) |

| Phase B | | |
|---|---|---|
| Isopropylstearat | Isopropyl Stearate | 9,14 % (3) |
| Cetylpalmitat | Cetyl Palmitate | 0,53% (1) |
| Ewalin 1751 | Petrolatum | 0,20 % (4) |
| Parfümöl Elegance # 79228 D MF | Parfum | 0,20 % (5) |
| Propyl-4-hydroxybenzoat | Propylparaben | 0,10 % (1) |

### Herstellung:

Bestandteile der Phase A zusammen geben und vormischen. Anschließend die Pudermischung unter Rühren tropfenweise mit der geschmolzenen Phase B versetzen. Die Puder werden bei 40 - 50 bar gepresst.

### Bezugsquellen:

(1) Merck KGaA
(2) Südstärke GmbH
(3) Cognis GmbH
(4) H. Erhard Wagner GmbH
(5) Haarmann & Reimer GmbH

### Beispiel A9: Loose Eye Powder

| Phase A | | |
|---|---|---|
| Fünfschichtpigment | Silica, Cl77891 (Titanium dioxide), | |
| gemäß Beispiel 1 | Mica,Tin oxide | 50,00 % (1) |

| Phase B | | |
|---|---|---|
| Micronasphere® M | Mica, Silica | 5,00 % (1) |
| Talkum | Talc | 30,00 % (1) |
| Weißer Ton (gestrichen) | Kaolin | 8,00 % (1) |
| Magnesiumstearat | Magnesium Stearate | 2,00 % (1) |

| Phase C | | |
|---|---|---|
| Ceraphyl 368 | Ethylhexyl Palmitate | 4,90 % (2) |
| Propyl-4-hydroxybenzoat | Propylparaben | 0,10 % (1) |

### Herstellung:

Alle Bestandteile der Phase B werden zusammen eingewogen, gut gemischt und bei 100 µm gesiebt. Anschließend Phase A zugeben und wieder mischen. Die Masse wird unter Rühren tropfenweise mit Phase C versetzt.

### Bezuqsauellen:

(1) Merck KGaA/Rona®
(2) ISP Global Technologies

### Beispiel A10: Creme Mascara (O/W)

| Phase A | | |
|---|---|---|
| Mica Black | Cl 77499 (Iron Oxides), Mica, Cl 77891 (Titanium Dioxide) | 10,00 % (1) |
| FÜnfschichtpigment gemäß Beispiel 2 | Silica, Cl77891 (Titanium dioxide), Mica, Tin oxide | 5,00 % (1) |

| Phase B | | |
|---|---|---|
| Stearinsäure | Stearic Acid | 8,00 % (1) |
| Bienenwachs gebleicht | Cera Alba(Beeswax) | 6,00 % (1) |
| Carnaubawachs 2442 L | Copernicia Cerifera(Carnauba Wax) | 4,00 % (2) |
| Eutanol G | Octyldodecanol | 3,00 % (3) |
| Arlacel 83 V | Sorbitan Sesquioleate | 2,00 % (4) |
| Propyl-4-hydroxybenzoat | Propylparaben | 0,10 % (1) |
| RonaCare™ Tocoherolacetat | Tocopheryl Acetate | 0,50 % (1) |

| Phase C | | |
|---|---|---|
| Wasser, demineralisiert | Aqua (Water) | 50,84 % |
| Triethanolamin reinst | Triethanolamine | 2,30 % (1) |
| Water Soluble Shellac SSB 63 | Shellac | 8,00 % (5) |
| Methyl-4-hydroxybenzoat | Methylparaben | 0,25 % (1) |
| RonaCare™ Biotin | Biotin | 0,01 % (1) |

### Herstellung:

Alle Bestandteile der Phase B zusammen bei ca. 80 °C aufschmelzen, rühren bis alles geschmolzen ist. Die Effektpigmente der Phase A einrühren. Shellac der Phase C im Wasser lösen, auf 75 °C erwärmen. Die restlichen Bestandteile der Phase C zugeben, lösen. Bei 75 °C langsam unter Rühren die Phase C zu Phase A/B geben, 2 min homogenisieren.
Die Masse unter Rühren auf Raumtemperatur abkühlen.

### Bezugsguellen:

(1) Merck KGaA/Rona®
(2) Kahl & Co.
(3) Cognis GmbH
(4) Uniqema
(5) Paroxite Ltd.

### Beispiel A11: Nagellack

| Phase A | | |
|---|---|---|
| Fünfschichtpigment | Silica, Cl77891 (Titanium dioxide), | |
| gemäß Beispiel 1 | Mica,Tin oxide | 2,00 % (1) |
| Thixotrope Nagellack-Base 1348 | Toluene, Ethyl Acetate, Butyl Acetate, Nitrocellulose, Tosylamide/Formaldehyde Resin, Dibutyl Phthalate, Isopropyl Alcohol, Stearalkonium Hectorite, Camphor, Acrylates Copolymer, Benzophenone-1 | 98,00 % (2) |
| Farbdispersion mit Nitrocellulose Lack (q.s.) | | |

### Herstellung:

Das Fünfschichtpigment wird zusammen mit der Lackbase eingewogen, gut mit einem Spatel von Hand vermischt und anschließend 10 min bei 1000 Upm gerührt.

### Bezugsquellen:

(1) Merck KGaA/Rona®
(2) International Lacquers S. A.

### Beispiel A12: Shampoo

| Phase A | | |
|---|---|---|
| Fünfschichtpigment gemäß Beispiel 2 | Silica,Cl77891 (Titanium dioxide), Mica,Tin oxide | 0,05 % (1) |
| Carbopol ETD 2020 | Acrylates/C10-30 Alkyl Acrylate Crosspolymer | 0,90 % (2) |
| Wasser, demineralisiert | Aqua(Water) | 59,90 % |

| Phase B | | |
|---|---|---|
| Triethanolamin reinst | Triethanolamine | 0,90 % (1) |
| Wasser, demineralisiert | Aqua (Water) | 10,00 % |

| Phase C | | |
|---|---|---|
| Plantacare 2000 UP | Decyl Glucoside | 20,00 % (3) |
| Texapon ASV | Magnesium Oleth Sulfate, Sodium Oleth Sulfate, Magnesium Laureth-8 Sulfate, Sodium Laureth-8 Sulfate, Magnesium Laureth Sulfate, Sodium Laureth Sulfate | 8,00 % (3) |
| Bronidox L | Propylene Glycol, 5-Bromo-5-Nitro-1,3-Dioxane | 0,20 % (3) |
| Parfümöl Everest 79658 SB | Parfum | 0,05 % (4) |

### Herstellung:

Für Phase A das Fünfschichtpigment in das Wasser einrühren. Mit einigen Tropfen Citronensäure (10%ig) ansäuern um die Viskosität zu vermindern und das Carbopol unter Rühren langsam einstreuen. Nach vollständiger Lösung langsam Phase B zugeben. Nacheinander werden nun die Bestandteile der Phase C zugegeben.

### Bezugsquellen:

(1) Merck KGaA
(2) BF Goodrich GmbH
(3) Cognis GmbH
(4) Haarmann & Reimer GmbH

### Beispiel A13: Sparkling Body Cream (O/W)

| Phase A | | |
|---|---|---|
| Fünfschichtpigment | Silica, Cl77891 (Titanium dioxide), | |
| gemäß Beispiel 2 | Mica,Tin oxide | 3,00 % (1) |
| Carbopol ETD 2001 | Carbomer | 0,60 % (2) |
| Citronensäure Monohydrat | Citric Acid | (1) |
| Wasser, demineralisiert | Aqua(water) | 40,00 % |

| Phase B | | |
|---|---|---|
| RonaCare™ Allantoin | Allantoin | 0,20 % (1) |
| 1,2-Propandiol | Propylene Glycol | 3,00 % (1) |
| Euxyl K 400 | Phenoxyethanol, Methyldibromo-Glutaronitrile | 0,10 % (3) |
| Chemag 2000 | Imidazolidinyl Urea | 0,30 % (4) |
| Methyl-4-hydroxybenzoat | Methylparaben | 0,15 % (1) |
| Wasser, demineralisiert | Aqua (Water) | 27,65 % |

| Phase C | | |
|---|---|---|
| Hostaphat KL 340 N | Dilaureth-4 Phosphate | 3,00 % (5) |
| Cetylalkohol | Cetyl Alcohol | 2,00 % (1) |
| Paraffin flüssig | Paraffinum Liquidum (Mineral Oil) | 10,00 % (1) |
| Cetiol V | Decyl Oleate | 6,00 % (6) |
| Propyl-4-hydroxybenzoat | Propylparaben | 0,05 % (1) |

| Phase D | | |
|---|---|---|
| Triethanolamin | Triethanolamine | 0,35 % (1) |
| Wasser, demineralisiert | Aqua (Water) | 3,50 % |

| Phase E | | |
|---|---|---|
| Parfümöl 72979 | Parfum | 0,10 % (7) |

### Herstellung:

Das Fünfschichtpigment im Wasser der Phase A dispergieren. Eventuell mit einigen Tropfen Citronensäure ansäuern, um die Viskosität zu vermindern. Carbopol unter Rühren einstreuen. Nach vollständiger Lösung die vorgelöste Phase B langsam einrühren. Phase A/B und Phase C auf 80 °C erhitzen, Phase C in Phase A/B einrühren, homogenisieren, mit Phase D neutralisieren, nochmals homogenisieren und unter Rühren abkühlen. Bei 40 °C Parfümöl zugeben, unter Rühren auf Raumtemperatur abkühlen.

### Bezugsquellen:

(1) Merck KGaA
(2) BF Goodrich GmbH
(3) Schülke & Mayr GmbH
(4) Chemag AG
(5) Clariant GmbH
(6) Cognis GmbH
(7) Haarmann & Reimer GmbH

### Beispiel A14: Kunststoff

Den Kunststoffgranulaten Polypropylen PP Stamylan PPH10 (Fa. DSM) bzw. Polystyrol 143E (Fa. BASF) werden jeweils
a) 1 % Pigment aus Beispiel 1
b) 1 % Pigment aus Beispiel 2
c) 1 % Pigment aus Beispiel 1 und 0,1 % PV-Echtblau B2G01 (Pigment Blue 15,3)
zugeben.
Das pigmentierte Granulat wird anschließend auf einer Spritzmaschine zu Stufenplättchen verarbeitet.

### Beispiel A15: Druckfarbe

Das Pigment wird bei 600 Upm in das lösemittelhaltige Bindemittel eingerührt und die Druckfarben werden anschließend auf Schwarz-Weiß-Karten aufgerakelt.

| Farbe Nr. 1: | |
|---|---|
| 88,0 g | Gebr. Schmidt 95 MB 011 TW |
| 10,0 g | Pigment aus Beispiel 1 |
| 2,0 g | Gebr. Schmidt 95 MB 022-TW (Grün) |

| Farbe Nr. 2: | |
|---|---|
| 88,0 g | Gebr. Schmidt 95 MB 011 TW |
| 10,0 g | Pigment aus Beispiel 2 |
| 2,0 g | Gebr. Schmidt 95 MB 022-TW (Grün) |

### Beispiel A16: Autolack

| | |
|---|---|
| 2,00 g | Pigment aus Beispiel 1 |
| 1,50 g | Heliogenblau L 6930 |
| 0,20 g | Hostapermgrün 8G |
| 0,05 g | Farbruß FW 200 |
| 66,60 g | Basislack (A4) MP-System (FK = 19 %) |
| 29,65 g | Verdünnermischung |

## Patentansprüche

1. Fünfschichtpigmente auf der Basis von mehrfach beschichteten plättchenförmigen Substraten, die mindestens eine Schichtenfolge aus
(A) einer Schicht aus SnO₂ mit einer Schichtdicke von 0,1-50 nm,
(B) einer Schicht aus TiO₂ in der Rutilmodifikation mit einer Schichtdicke von 10-800 nm,
(C) einer farblosen Beschichtung mit einem Brechungsindex n ≤ 1,8 mit einer Schichtdicke von 20-800 nm,
(D) einer Schicht aus SnO₂ mit einer Schichtdicke von 0,1-50 nm,
(E) einer Schicht aus TiO₂ in der Rutilmodifikation mit einer Schichtdicke von 10-800 nm,
und optional
(F) einer äußeren Schutzschicht
enthält.

2. Fünfschichtpigmente nach Anspruch 1, **dadurch gekennzeichnet, dass** es sich bei den plättchenförmigen Substraten um natürlichen oder synthetischen Glimmer, Glas-, Al₂O₃-, SiO₂- oder TiO₂-Plättchen handelt.

3. Fünfschichtpigmente nach Anspruch 1 oder 2, **dadurch gekennzeichnet, dass** die Schicht (C) aus SiO₂, Al₂O₃, AIO(OH), B₂O₃, MgF₂, MgSiO₃ oder deren Gemische besteht.

4. Fünfschichtpigmente nach einem der Ansprüche 1 bis 3, **dadurch gekennzeichnet, dass** sie zu Erhöhung der Licht-, Temperatur- und Wetterstabilität eine äußere Schutzschicht (F) aufweisen.

5. Verfahren zur Herstellung der Fünfschichtpigmente nach einem der Ansprüche 1 bis 4, **dadurch gekennzeichnet, dass** die Beschichtung der Substrate nasschemisch durch hydrolytische Zersetzung von Metallsalzen in wässrigem Medium erfolgt.

6. Verwendung der Fünfschichtpigmente nach Anspruch 1 in Farben, Lacken, Pulverlacken, Druckfarben, Sicherheitsdruckfarben, Kunststoffen, keramischen Materialien, Gläsern, als Dotierstoff für die Lasremarkierung von Papieren und Kunststoffen, in kosmetischen Formulierungen und zur Herstellung von Pigmentpräparationen und Trockenpräparaten.

7. Pigmentpräparationen enthaltend ein oder mehrere Bindemittel, optional ein oder mehrere Additive und ein oder mehrere Fünfschichtpigmente gemäß Anspruch 1.

8. Pigmentpräparationen nach Anspruch 7, **dadurch gekennzeichnet, dass** es sich um Trockenpräparate handelt.

9. Trockenpräparate nach Anspruch 8, **dadurch gekennzeichnet, dass** sie 0 bis 8 Gew.% an Wasser und/oder eines Lösemittels bzw. Lösemittelgemisches enthalten.

10. Trockenpräparate nach Anspruch 8 oder 9, **dadurch gekennzeichnet, dass** es sich um Pellets, Granulate, Chips oder Brickets handelt.

11. Hydrophile oder hydrophile kosmetische Formulierungen enthaltend ein oder mehrere Fünfschichtpigmente nach Anspruch 1.
